# EUROPEAN PATENT APPLICATION

(11) **EP 4 455 156 A1**
(43) Date of publication of application: **30.10.2024**
(21) Application number: 22909976.7
(22) Date of filing: 20.12.2022
(51) Int. Cl.: C07K 5/037, C07K 7/02, C07K 1/02

(54) **PREPARATION METHOD FOR OXIDIZED GLUTATHIONE AND CRYSTAL FORM AND IMPURITY THEREOF**

(30) Priority: 21.12.2021 CN 202111573909; 08.12.2022 CN 202211572513
(71) Applicant: Shenyang Xingqi Pharmaceutical Co., Ltd., Shenyang, Liaoning 110167 (CN)
(72) Inventor: LIU, Jidong, Shenyang, Liaoning 110163 (CN); YANG, Qiang, Shenyang, Liaoning 110163 (CN); LEI, Yu, Shenyang, Liaoning 110163 (CN); SHEN, Jinsong, Shenyang, Liaoning 110163 (CN)
(74) Representative: Casalonga
(86) International application number: PCT/CN2022/140269
(87) International publication number: WO 2023/116664

(57) **Abstract**

Provided is a method for preparing oxidized glutathione and a new crystal form and impurity thereof, comprising the following steps: using dimethyl sulfoxide (DMSO) as an oxidizing agent to oxidize reduced glutathione to crude oxidized glutathione; and recrystallizing and refining in purified water to obtain high-purity heptahydrate crystals of oxidized glutathione.

## Description

### TECHNICAL FIELD

The present disclosure belongs to the field of chemical synthesis, and particularly relates to a preparation method of oxidized glutathione and its heptahydrate crystal form and impurities.

### BACKGROUND

Oxidized glutathione has similar effects to reduced glutathione, but oxidized glutathione is more stable than reduced glutathione, and thus can be used as an active ingredient in health foods, medicines, cosmetics and other products in replace of reduced glutathione.

The currently reported methods for preparing oxidized glutathione are all from the oxidation of reduced glutathione, for example:
Route 1: Oxidation by hydrogen peroxide (Chinese Journal of Pharmaceuticals, 2013, 44, 265). The method comprises the following steps: dissolving reduced glutathione in water, adjusting the obtained solution to a suitable pH value, and oxidizing glutathione with hydrogen peroxide as an oxidizing agent to prepare oxidized glutathione. The disadvantages of this method are that although the speed of the hydrogen peroxide oxidation reaction is relatively fast, the reaction is also relatively intense, so the reaction conditions are relatively harsh, and the process parameters such as temperature, pH and amount of hydrogen peroxide in the reaction system need to be strictly controlled. Otherwise, more peroxidation or degradation impurities will appear, affecting the purity and yield of the product. In addition, the oxidizing agent, hydrogen peroxide, is a hazardous chemical in China that can easily produce explosives, and thus the use of hydrogen peroxide is controlled. In addition, hydrogen peroxide is prone to self-degradation and needs to be stored in a sealed container at low temperature.
Route 2: Catalysis by arginine (RSC Adv., 2014, 4, 33399-33407). The method comprises the following steps: dissolving reduced glutathione in water, and using oxygen gas as an oxidizing agent and arginine as a catalyst. The reaction produces no waste, and is green and environmentally friendly. Moreover, oxygen gas and arginine are relatively easy to obtain, and thus raw materials are relatively convenient to obtain. The disadvantages of this method are that: arginine needs to be used as a catalyst during the reaction process, and thus residual arginine is likely to remain during the work-up process; and the reaction needs to be heated to 50°C, and thus the product, i.e., oxidized glutathione, is prone to partial degradation and racemization under high temperature conditions, resulting in reduced yield and decreased purity.
Route 3: Catalysis by enzyme (Japanese Patent Application Publication No. 5-146279). This method is a biological enzyme catalysis method, which requires the use of a specific biological oxidase as a catalyst and uses air to oxidize the reduced glutathione aqueous solution to obtain oxidized glutathione. The disadvantages of this method are that: enzymes are relatively difficult to obtain and preserve, and are not as good as commonly used chemical reagents. Moreover, after the reaction is completed, the enzyme needs be separated from the reaction solution, which requires specific process technology and equipment. The existing equipment of general chemical raw material pharmaceutical companies may not be applicable.
Route 4: Diethyl bromomalonate method (Chem. Pharm. Bull. 1986, 34, 486-495). The method comprises the following steps: dissolving reduced glutathione in an alkaline water/ethanol solution, then adding a solution of diethyl bromomalonate in ethanol dropwise, and reacting at -16°C for 1 hour. The disadvantages of this method are that: the oxidizing agent uses diethyl bromomalonate, which is relatively expensive, diethyl bromomalonate generates more by-products, and the operation is complicated. The reaction process requires a low temperature of -1 6°C, which is relatively unsuitable for industrialization.

In addition, the crystal forms of oxidized glutathione prepared by the above method mainly include non-crystalline amorphous form (CN102858792A), monohydrate crystal form (Japanese Patent No. 4401775), hexahydrate crystal form (CN102869674A) and octahydrate crystal form (International Union of Crystallography, pp538, 1999). In these forms, the poor water solubility of the non-crystalline amorphous form limits its application in the pharmaceutical industry; the monohydrate crystal form has poor crystal separation ability because it is a needle-shaped crystal and is easy to agglomerate, and the impurities therein are difficult to remove; during the crystallization process of the hexahydrate crystal form, pH needs to be adjusted, and the crystallization time is as long as more than ten hours, so the operation is relatively cumbersome and it is difficult to obtain crystals; and the octahydrate crystal form has uneven water content, poor stability, and require a long time of up to 3-4 days to obtain crystals. Moreover, the existing processes for oxidized glutathione have not achieved large-scale industrial production. The main reason is that the mildness (reducing product degradation and racemization) and economy of the reaction are difficult to achieve at the same time. An oxidation and crystallization system that is mild, economical, readily available, and has few by-products is needed to achieve excellent process stability.

In addition, through the detection by liquid chromatography and the structural confirmation by mass spectrometry, oxidized glutathione contains the following three impurities:

| | |
|---|---|
| Impurity A | 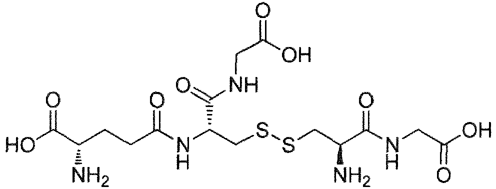 |
| Impurity B | 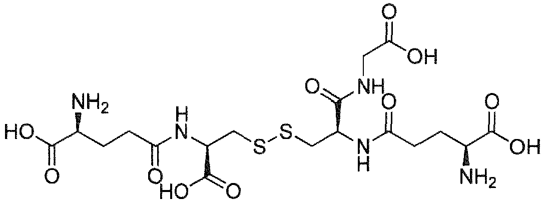 |
| Impurity C | 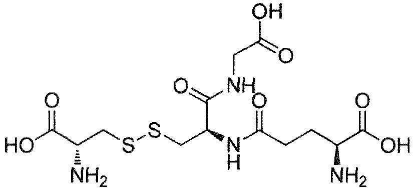 |

There are few reports on the preparation methods of these three impurities, and the raw materials and reagents used are not available in the market. Therefore, these preparation methods lack practical value.

There are no literature reports on the purification process of oxidized glutathione. Because polypeptide compounds are easily hydrolyzed, racemized, and biodegraded, the commonly used purification methods are separation by ion exchange resin chromatography or preparative liquid chromatography. The above methods have high solvent loss and high production cost.

In summary, there is a need in the art for a method for synthesizing oxidized glutathione that has low cost, mild reaction condition, and high product purity, and is suitable for industrial production.

### SUMMARY

Compared with the existing methods, the synthesis method of oxidized glutathione provided by the present disclosure can solve the above problems.

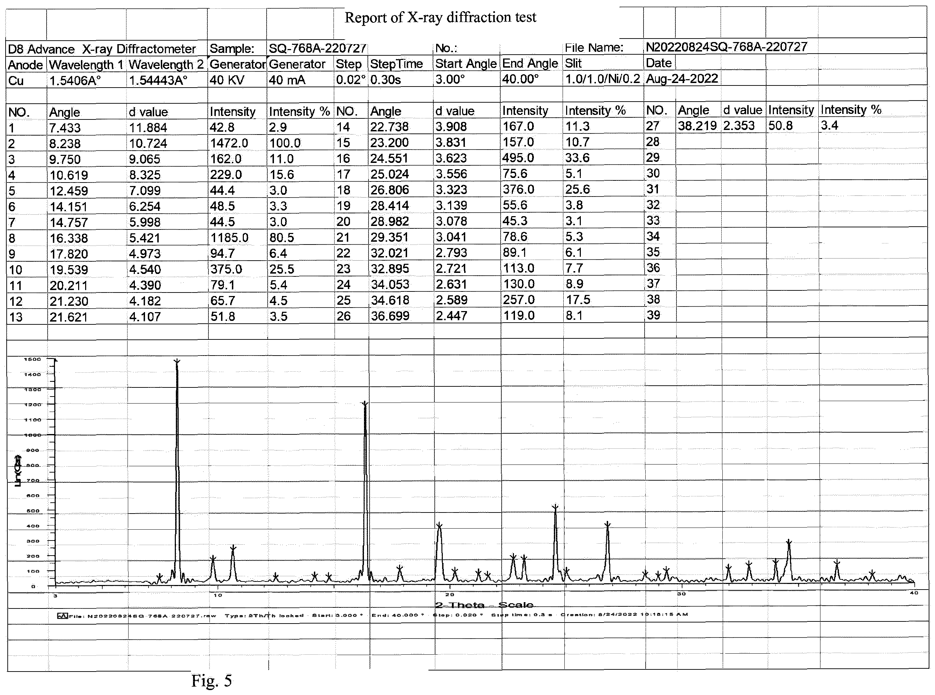

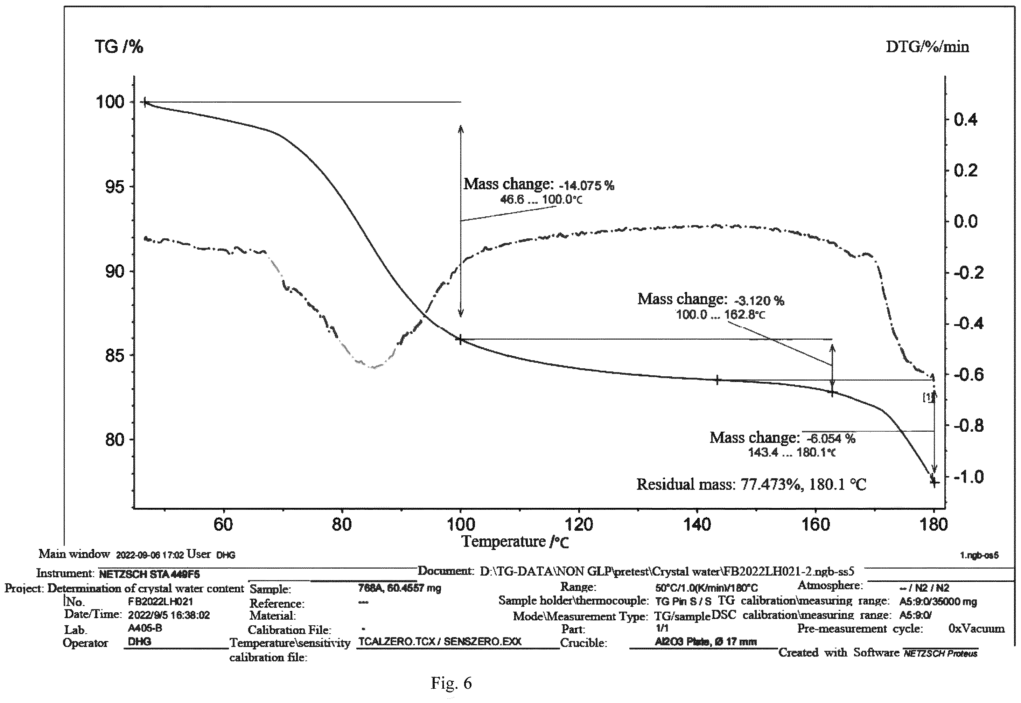

Specifically, the present disclosure relates to the following embodiments:
1. A crystal of a heptahydrate of a compound of formula (I): 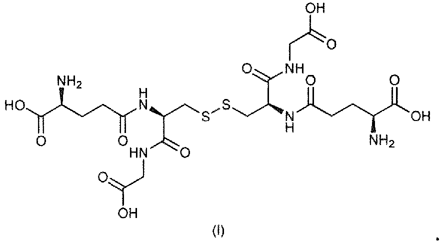
2. The crystal of embodiment 1, which is characterized by an X-ray powder diffraction pattern obtained using CuKa radiation having at least the following characteristic peaks at °2θ: 8.238±0.2, 16.338±0.2 and 24.551±0.2.
3. The crystal of embodiment 2, which is characterized by an X-ray powder diffraction pattern obtained using CuKa radiation having at least the following characteristic peaks at °2θ: 10.619±0.2, 19.539±0.2, 26.806±0.2 and 34.618±0.2.
4. The crystal of embodiment 3, which is characterized by an X-ray powder diffraction pattern obtained using CuKa radiation having at least the following characteristic peaks at °2Θ: 9.750±0.2, 22.738±0.2 and 23.200±0.2.
5. The crystal of embodiment 1, which is characterized by having an X-ray powder diffraction pattern substantially as shown in Fig. 5.
6. The crystal of any one of embodiments 1 to 5, which is further characterized by having a melting point of 169 ± 2 °C.
7. The crystal of any one of embodiments 1 to 6, which is further characterized by a weight loss of about 14 ± 1% at 50-100 °C, and a weight loss of about 3%±1% at 100-160 °C in thermogravimetric analysis.
8. The crystal of any one of embodiments 1 to 7, which is further characterized by having a thermogravimetric diagram as shown in Fig. 6.
9. A method for preparing a compound of formula (1), 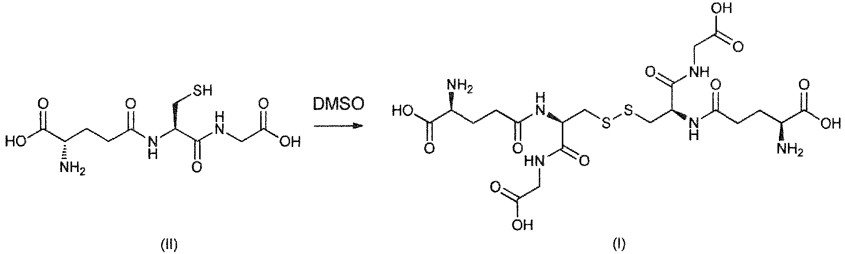 comprising oxidizing the compound of formula (II) with DMSO to obtain the compound of formula (I).
10. The method of embodiment 9, wherein the molar ratio of DMSO to the compound of formula (II) is 2:1 to 25:1, alternatively 2.5:1 to 5:1, yet alternatively 2:1, 2.5:1, 3:1, 3.5:1, 4:1, 4.5:1 or 5:1.
11. The method of embodiment 9 or 10, wherein a polar solvent is additionally added to the reaction to promote the dissolution of the compound of formula (II); alternatively, the polar solvent is selected from the group consisting of water, formamide, trifluoroacetic acid, acetonitrile, DMF, hexamethylphosphoramide, methanol, ethanol, acetic acid, isopropanol, pyridine, tetramethylethylenediamine, acetone, triethylamine, n-butanol, dioxane, tetrahydrofuran, methyl formate, tributylamine, methyl ethyl ketone, ethyl acetate, chloroform, trioctylamine, dimethyl carbonate and diethyl ether, or a compound thereof, yet alternatively water.
12. The method of embodiment 11, wherein the ratio of the polar solvent to the compound of formula (II) is 200 to 2000 mL, alternatively 250 mL to 1000 mL, yet alternatively 250 mL, 300 mL, 500 mL, 750 mL or 1000 mL of the polar solvent per 100 g of the compound of formula (II).
13. The method of any one of embodiments 9 to 12, wherein the pH of the reaction is adjusted to 2 to 9, alternatively 3 to 7, yet alternatively 5 to 7, such as 5, 5.5, 6, 6.5, 7, 8 or 9.
14. The method of any one of embodiments 9 to 13, wherein the reaction is carried out at -10 °C to 60°C, alternatively 5 °C to 30 °C, such as 25 °C, 40 °C or room temperature.
15. The method of any one of embodiments 9 to 14, wherein the reaction is carried out for 5 to 60 h, alternatively 10 to 48 h, such as 10 h, 15 h, 20 h, 25 h, 30 h, 35h, 40h or 45h.
16. The method of any one of embodiments 9 to 15, wherein after the reaction is completed, the pH is adjusted to the isoelectric point of oxidized glutathione (pH 2.75 to 2.90), and the stirring crystallization is performed for at least 5 h, alternatively at least 10 hours.
17. The method of embodiment 16, wherein the stirring crystallization is performed at 5°C to 40 °C, alternatively 5°C, 10 °C, 15 °C, 20 °C or room temperature.
18. The method of any one of embodiments 9 to 17, wherein the purity of the compound of formula (I) obtained is ≥ 98%, alternatively ≥ 98.5%, alternatively ≥ 99%, alternatively ≥ 99.7%, alternatively ≥ 99.8%, alternatively ≥ 99.9%; and wherein the total content of impurity A, impurity B and impurity C in the product is less than 1 %, alternatively less than 0.5%, alternatively less than 0.3%, alternatively less than 0.1%,

| | |
|---|---|
| Impurity A | 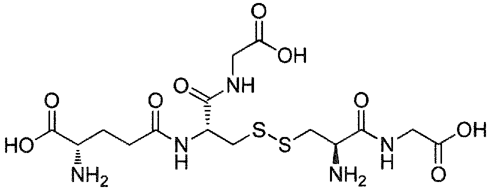 |
| Impurity B | 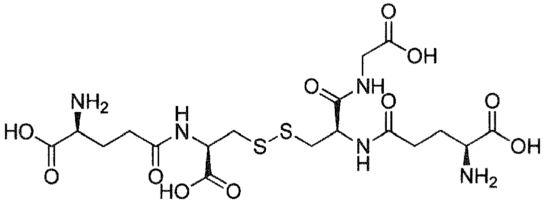 |
| Impurity C | 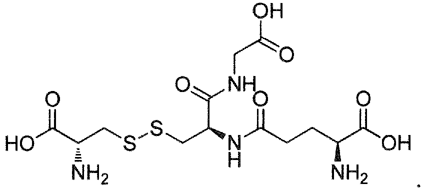 |

19. A method for refining the oxidized glutathione of any one of embodiments 9 to 18, comprising:
1) dissolving a crude oxidized glutathione in purified water, wherein the amount of purified water is 3 to 5 times the mass of the crude oxidized glutathione;
2) filtering the solution while hot after the dissolution;
3) cooling the filtrate to 10 to 25°C, alternatively 12 to 20°C for crystallization.

20. The method of embodiment 19, wherein in step 1), the temperature of the purified water is 35°C to 55°C, alternatively 40°C to 50°C; and optionally, ferrous chloride solution is added to remove residual raw materials, alternatively, 100 mL of 5% ferrous chloride solution is added per kilogram of oxidized glutathione.
21. The method of embodiment 19 or 20, wherein in step 3), the crystallization time of recrystallization is 3 to 10 hours, alternatively 4 to 6 hours; optionally, gradient cooling is adopted, and the cooling rate is 10°C per hour.
22. A method for preparing the crystal of oxidized glutathione heptahydrate according to any one of embodiments 1 to 8, comprising recrystallizing the oxidized glutathione prepared according to any one of embodiments 9 to 21 in purified water; optionally, the method comprises the following steps:
1) dissolving the oxidized glutathione in purified water and stirring until dissolved;
2) filtering the solution while hot after the dissolution, and then gradually cooling down to the target temperature;
3) crystallization under controlled temperature.

23. The method of embodiment 22, wherein in step 1), 2L to 6 L, alternatively 3 L to 6 L, yet alternatively 3 L to 5 L, still alternatively 4 L of purified water is used per kilogram of oxidized glutathione.
24. The method of embodiment 22 or 23, wherein in step 1), the temperature of the purified water is 40-60°C, alternatively 40-50 °C, yet alternatively 50 °C during the dissolution process.
25. The method of any one of embodiments 22 to 24, wherein in step 1), ferrous chloride solution is added to better remove residual raw materials, optionally, 100 mL of 5% ferrous chloride solution is added per kilogram of oxidized glutathione.
26. The method of any one of embodiments 22 to 25, wherein in step 2), the cooling gradient is 5-25 °C/h, alternatively 5-20 °C/h, alternatively 5-15 °C/h, yet alternatively 10 °C/h.
27. The method of any one of embodiments 22 to 26, wherein in step 2), the target temperature is 5-25 °C, alternatively 10-25 °C, yet alternatively 15-25 °C.
28. The method of any one of embodiments 22 to 27, wherein in step 3), the temperature is controlled at 5-25 °C, alternatively 10-25 °C, yet alternatively 15-25 °C.
29. The method of any one of embodiments 22 to 28, wherein in step 3), the crystallization time is 6 to 12 hours, alternatively 6 to 8 hours.
30. A compound selected from:

| | |
|---|---|
| Impurity A | 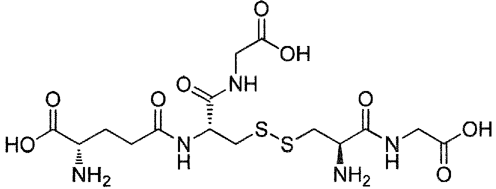 |
| Impurity B | 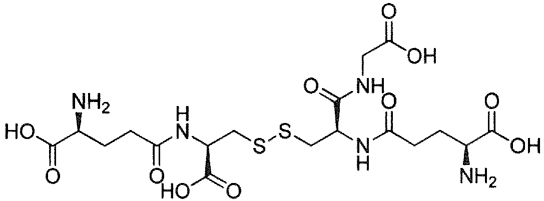 |
| Impurity C | 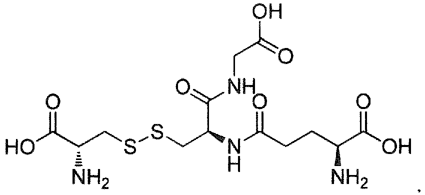 |

31. A method for preparing impurity A in embodiment 30, 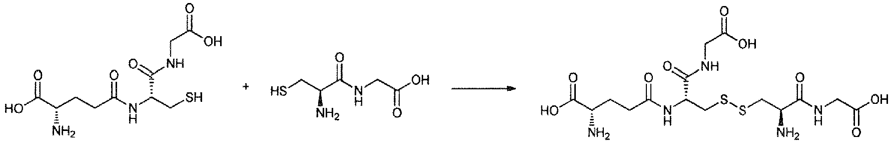 comprising oxidizing reduced glutathione and Cys-Gly in a molar ratio of 1:1 with DMSO.
32. The method of embodiment 31, wherein the amount of DMSO is 2 to 10 equivalents, alternatively 3 to 5 equivalents.
33. The method of embodiment 31 or 32, comprising the steps of:
1) condensing Boc-Cys(Trt)-OH and glycine tert-butyl ester in a molar ratio of 1:1 to 1:3 under the catalysis of 1.5 to 4 equivalents of a condensation agent (such as HATU, HBTU, PyBOP, DEPBT, etc., alternatively HBTU and DEPBT) and 2 equivalents of an organic tertiary amine (such as N,N-diisopropylethylamine and triethylamine, alternatively N,N-diisopropylethylamine), wherein the reaction is carried out in DMF or dichloromethane;
2) removing Trt, Boc and tBu protective groups with trifluoroacetic acid to obtain Cys-Gly;
3) oxidizing reduced glutathione and Cys-Gly in a molar ratio of 1:1 with 3 to 5 equivalents of DMSO.

34. A method for preparing impurity B in embodiment 30, 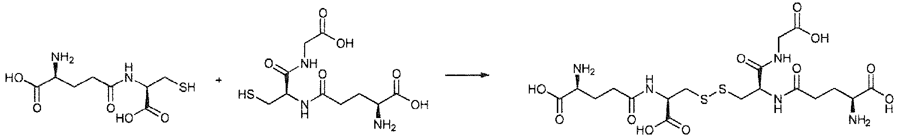 comprising oxidizing reduced glutathione and Glu-Cys in a molar ratio of 1:1 with DMSO.
35. The method of embodiment 34, wherein the amount of DMSO is 2 to 10 equivalents, alternatively 3 to 5 equivalents.
36. The method of embodiment 34 or 35, comprising the steps of:
1) condensing Boc-Glu-OtBu and H-Cys(Trt)-OtBu in a molar ratio of 1:1 to 1:2 under the catalysis of 1.5 to 4 equivalents of a condensation agent (such as HATU, HBTU, PyBOP, DEPBT, etc., alternatively HBTU and DEPBT) and 2 equivalents of an organic tertiary amine (such as N,N-diisopropylethylamine and triethylamine, alternatively N,N-diisopropylethylamine),
2) removing Boc, Trt, and tBu protective groups with trifluoroacetic acid to obtain Glu-Cys;
3) oxidizing reduced glutathione and Glu-Cys in a molar ratio of 1:1 with 3 to 5 equivalents of DMSO.

37. A method for preparing impurity C in embodiment 30, comprising oxidizing reduced glutathione and cysteine in a molar ratio of 1:1 with DMSO.
38. The method of embodiment 37, wherein the amount of DMSO is 2 to 10 equivalents, alternatively 3 to 5 equivalents.

### Beneficial effects of the present disclosure:

The crude oxidized glutathione obtained by this method does not contain peroxidation impurities, and has a relatively low content of hydrolysis impurities; and no other solid by-products are generated; after analyzing and synthesizing the structures of other impurities, the basis and guarantee for the subsequent quality control of oxidized glutathione are provided.

The inventors accidentally noticed that during the purification process of oxidized glutathione, adding a small amount of ferrous chloride aqueous solution can unexpectedly significantly improve the purification effect and increase the purity of the product; and crystals of oxidized glutathione heptahydrate can be obtained by recrystallization in purified water under certain conditions.

### BRIEF DESCRIPTION OF DRAWINGS

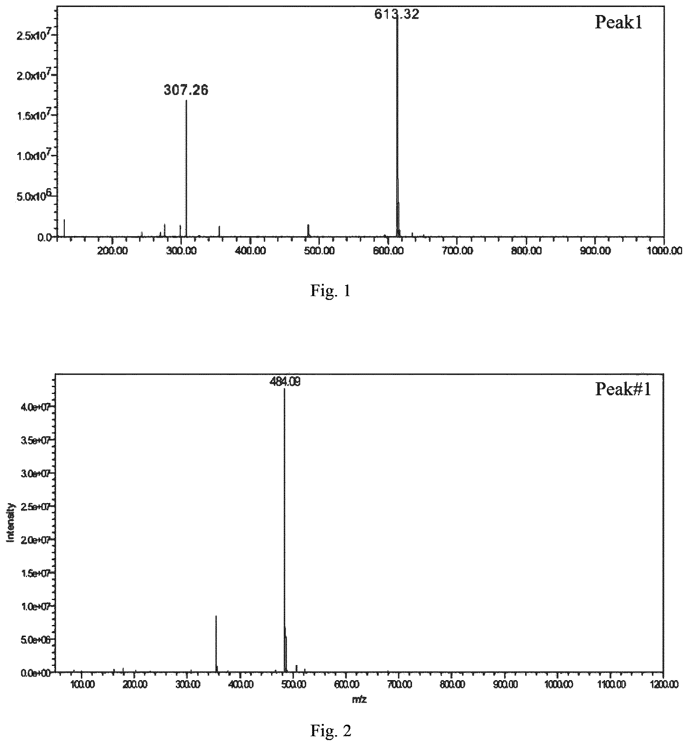

Fig. 1 is an ESI-MS diagram of oxidized glutathione;
Fig. 2 is an ESI-MS diagram of impurity A of oxidized glutathione;

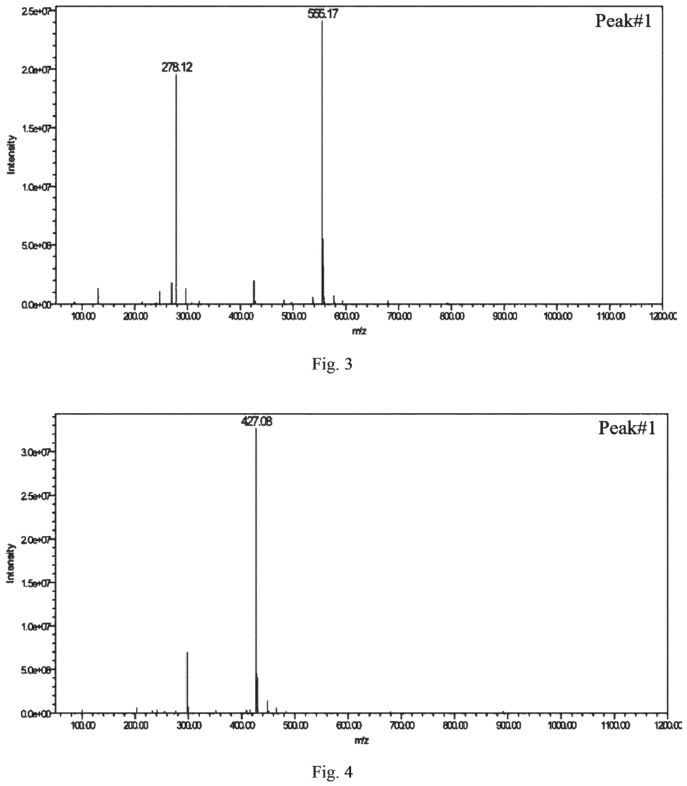

Fig. 3 is an ESI-MS diagram of impurity B of oxidized glutathione;
Fig. 4 is an ESI-MS diagram of impurity C of oxidized glutathione;
Fig. 5 is an X-ray powder diffraction pattern of crystals of oxidized glutathione heptahydrate;
Fig. 6 is TG and DSC diagrams of crystals of oxidized glutathione heptahydrate.

### DETAILED DESCRIPTION

### Definition

"Polar solvent" refers to a solvent containing a polar group such as hydroxyl, carbonyl, carboxyl, etc., that is, the solvent molecules are polar molecules because the centers of gravity of positive and negative charges in the molecules do not coincide, which causes the molecules to become polar. The polar solvent is selected from the group consisting of water, formamide, trifluoroacetic acid, acetonitrile, DMF, hexamethylphosphoramide, methanol, ethanol, acetic acid, isopropanol, pyridine, tetramethylethylenediamine, acetone, triethylamine, n-butanol, dioxane, tetrahydrofuran, methyl formate, tributylamine, methyl ethyl ketone, ethyl acetate, chloroform, trioctylamine, dimethyl carbonate and diethyl ether, or a compound thereof.

"Isoelectric point" is the pH value at which a molecule has no surface charge. At the isoelectric point, because there is no mutual repulsion due to the same charge, the molecules are most unstable, have the lowest solubility, and can easily combine into larger aggregates quickly by electrostatic attraction, thereby precipitating out.

Water content is calculated as follows:
Water content = mass of water molecules * number of water molecules in a single crystal molecule / (mass of water molecules * number of water molecules in a single crystal molecule + molecular mass of oxidized glutathione) * 100%.

For example, the water content of monohydrate = 18.01*1/(18.01*1+612.63)*100%=2.86%, the water content of hexahydrate = 18.01*6/(18.01*6+612.63)=14.99%, the water content of heptahydrate = 18.01*7/(18.01*7+612.63) = 17.07%, and the water content of octahydrate = 18.01*8/(18.01*8+612.63) = 19.04 %.

The embodiments of the present disclosure will be clearly and completely described below with reference to the examples. Obviously, the described examples are only used to illustrate the present disclosure, but not used to limit the present disclosure. Based on the examples of the present disclosure, all other examples obtained by those of ordinary skill in the art without making creative efforts fall within the scope of protection of the present disclosure.

### Example

### (1) Synthesis of oxidized glutathione

### Example 1

1.0 kg of reduced glutathione and 10.0 L of water were added into a three-necked flask, and then 1.0 L of dimethyl sulfoxide was added into the three-necked flask. The mixture was reacted with stirring at room temperature for 48 hours. A solid was precipitated, and the solid was filtered and dried to give 950.2 g of crude oxidized glutathione, with a yield of 95.3% and a purity of 98.4%. ESI-MS [M + H]⁺ = 613.3.

### Example 2

1.0 kg of reduced glutathione and 3.0 L of water were added into a three-necked flask. The pH of the mixture was adjusted to 6 to 7 with NaOH, and then 500 mL of dimethyl sulfoxide was added. The mixture was stirred at room temperature for 10 hours. The pH was adjusted to the isoelectric point (2.75 to 2.90) and the mixture was stirred at 5°C for 10 hours for crystallization. The mixture was filtered. The filter cake was rinsed with ethanol, and dried to give 855.1 g of crude oxidized glutathione, with a yield of 86.0% and a purity of 99.3%. ESI-MS [M + H]⁺ = 613.3.

### Example 3

1.0 kg of reduced glutathione and 2.5 L of water were added into a three-necked flask. The pH was adjusted to 6.0 with NaOH, and then 450 mL of dimethyl sulfoxide was added into the three-necked flask. The mixture was stirred at 40°C for 24 hours. The pH was adjusted to the isoelectric point (2.75 to 2.90) and the mixture was crystallized at 8°C for 10 hours. The mixture was filtered. The filter cake was rinsed with ethanol, and dried to give 892.7 g of oxidized glutathione, with a yield of 89.5% and a purity of 99.0%. ESI-MS [M + H]⁺ = 613.3.

### Example 4

1.0 kg of reduced glutathione and 3.0 L of water were added into a three-necked flask. The pH was adjusted to 5.5 with NaOH, and then 550 mL of dimethyl sulfoxide was added into the three-necked flask. The mixture was stirred at 25°C for 48 hours. The pH was adjusted to the isoelectric point (2.75 to 2.90) and the mixture was crystallized at 5°C for 8 hours. The mixture was filtered. The filter cake was rinsed with ethanol, and dried to give 912.0 g of oxidized glutathione, with a yield of 91.5% and a purity of 99.2%. ESI-MS [M + H]⁺ = 613.3.

### Comparative Example 1: Oxidation by hydrogen peroxide

10 g of reduced glutathione and 30 mL of water were added into a three-necked flask. The pH was adjusted to 5.8 with NaOH, and 3.3 mL of 30% hydrogen peroxide was added into the three-necked flask in a water bath at room temperature. The mixture was reacted for 5 hours. Then the pH was adjusted to 3.0, and 50 mL of anhydrous ethanol was added to the system. The mixture was crystallized at 10°C for 10 hours, and then filtered. The filter cake was rinsed with ethanol, and dried to give 9.09 g of oxidized glutathione containing peroxide impurities, with a yield of 91.2% and a purity of 96.2%. ESI-MS [M + H]⁺ =613.3.

### (2) Refining of oxidized glutathione

### Comparative Example 2: Purification and refining by a conventional method

10 g of crude oxidized glutathione obtained in the examples of above (1) Synthesis of oxidized glutathione was dissolved in 200 mL of purified water. The sample was then injected into a strong acidic cation exchange resin, and eluted with purified water. The eluent was collected, and then a solid was precipitated by adding anhydrous ethanol to give 8.43 g of oxidized glutathione, with a one-step yield of 84.3% and a purity of 99.5%. The purity increase was 0.2%.

### Example 5

1.0 kg of crude oxidized glutathione obtained in the examples of above (1) Synthesis of oxidized glutathione was added to 4.0 L of purified water at 50°C, and 100 mL of 5% ferrous chloride solution was added. The mixture was stirred until the solid was dissolved. The mixture was filtered while hot, and then gradually cooled down to 25 °C, 10°C per hour. Finally, the mixture was controlled at a temperature of 20 °C to 25 °C, and crystallized for 8 hours. The mixture was filtered. The filter cake was rinsed with ethanol, and dried.

In other experimental examples, the parameters in the following tables were used and the above steps were performed sequentially.

### Exploration of precipitation time

**Table 1: Experimental examples of precipitation time exploration**

| | Example 5 | Example 6 | Example 7 | Example 8 | Example 9 |
|---|---|---|---|---|---|
| The ratio of crude product to purified water (kg:L) | 1:4 | 1:4 | 1:4 | 1:4 | 1:4 |
| Dissolution temperature (°C) | 50 | 50 | 50 | 50 | 50 |
| Cooling gradient (°C) | 10 | 10 | 10 | 10 | 10 |
| Crystallization holding temperature (°C) | 20-25 | 20-25 | 20-25 | 20-25 | 20-25 |
| Crystallization time (h) | 8 | 6 | 4 | 3 | 12 |

**Table 2: Results of each experimental example of precipitation time exploration**

| | Example 5 | Example 6 | Example 7 | Example 8 | Example 9 |
|---|---|---|---|---|---|
| Crystallization mass (g) | 855.5 | 843.2 | 696.8 | 648.4 | 838.6 |
| Yield (%) | 85.5 | 84.3 | 69.7 | 64.8 | 83.9 |
| Purity (%) | 99.9 | 99.9 | 99.9 | 99.9 | 99.7 |
| Water content (%) | 17.2 | 17.3 | 17.2 | 15.8 | 17.2 |

According to the above results, it can be seen that: when the crystallization time is too short, the obtained crystals are mixed crystals with unstable water content, and when the crystallization time is too long, impurities will precipitate, resulting in reduced product purity. Therefore, the crystallization time is alternatively 6-12 hours, alternatively 6-8 hours.

### Exploration of purified water multiple

**Table 3: Experimental examples of exploration of purified water multiple**

| | Example 10 | Example 11 | Example 5 | Example 12 | Example 13 | Example 14 |
|---|---|---|---|---|---|---|
| The ratio of crude product to purified water (kg:L) | 1:1 | 1:2 | 1:4 | 1:6 | 1:8 | 1:9 |
| Dissolution temperature (°C) | 50 | 50 | 50 | 50 | 50 | 50 |
| Cooling gradient (°C) | 10 | 10 | 10 | 10 | 10 | 10 |
| Crystallization holding temperature (°C) | 20-25 | 20-25 | 20-25 | 20-25 | 20-25 | 20-25 |
| Crystallization time (h) | 8 | 8 | 8 | 8 | 8 | 8 |

**Table 4: Results of each experimental example of exploration of purified water multiple**

| | Example 10 | Example 11 | Example 5 | Example 112 | Example 13 | Example 14 |
|---|---|---|---|---|---|---|
| Crystallization mass (g) | Not fully soluble | 916.5 | 855.5 | 834.4 | 669.4 | 528.3 |
| Yield (%) | - | 91.7 | 85.5 | 83.4 | 66.9 | 52.8 |
| Purity (%) | - | 99.7 | 99.9 | 99.9 | 99.9 | 99.9 |
| Water content (%) | - | 17.1 | 17.2 | 17.2 | 17.2 | 17.2 |

According to the above results, it can be seen that: when purified water is used to dissolve oxidized glutathione, the amount of water has a greater impact on the yield. When the amount of water is too much, the yield is too low. When the amount of water is too little, the product cannot be completely dissolved, or after dissolution and re-precipitation, the stirring effect is not good and the product purity is reduced. Therefore, the water amount is alternatively 2-6 times, alternatively 3-6 times, yet alternatively 3-5 times.

### Exploration of dissolution temperature

**Table 5: Experimental examples of solution temperature exploration**

| | Example 15 | Example 16 | Example 5 | Example 17 |
|---|---|---|---|---|
| The ratio of crude product to purified water (kg:L) | 1:4 | 1:4 | 1: 4 | 1:4 |
| Dissolution temperature (°C) | 30 | 40 | 50 | 60 |
| Cooling gradient (°C) | 10 | 10 | 10 | 10 |
| Crystallization holding temperature (°C) | 20-25 | 20-25 | 20-25 | 20-25 |
| Crystallization time (h) | 8 | 8 | 8 | 8 |

**Table 6: Results of each experimental example of dissolution temperature exploration**

| | Example 15 | Example 16 | Example 5 | Example 17 |
|---|---|---|---|---|
| Crystallization mass (g) | Not fully soluble | 842.5 | 855.5 | 857.3 |
| Yield (%) | - | 84.3 | 85.5 | 85.7 |
| Purity (%) | - | 99.9 | 99.9 | 99.8 |
| Water content (%) | - | 17.1 | 17.2 | 17.2 |

According to the above table, it can be seen that: when the temperature is higher than 40°C, all examples are soluble, but it is also found that when the temperature is higher than 60°C, racemization phenomenon will occur. Moreover, the higher the temperature and the longer the heating time, the more serious the racemization phenomenon. Considering the production energy consumption, the selected temperature is 40-60 °C, alternatively 40-50°C, yet alternatively 50°C.

### Exploration of crystallization holding temperature

**Table 7: Experimental examples of exploration of crystallization holding temperature**

| | Example 18 | Example 19 | Example 20 | Example 5 | Example 21 |
|---|---|---|---|---|---|
| The ratio of crude product to purified water (kg:L) | 1:4 | 1:4 | 1:4 | 1:4 | 1:4 |
| Dissolution temperature (°C) | 50 | 50 | 50 | 50 | 50 |
| Cooling gradient (°C) | 10 | 10 | 10 | 10 | 10 |
| Crystallization holding temperature (°C) | 5-10 | 10-15 | 15-20 | 20-25 | 25-30 |
| Crystallization time (h) | 8 | 8 | 8 | 8 | 8 |

**Table 8: Results of each experimental example of exploration of crystallization holding temperature**

| | Example 18 | Example 19 | Example 20 | Example 5 | Example 21 |
|---|---|---|---|---|---|
| Crystallization mass (g) | 900.6 | 877.2 | 867.2 | 855.5 | 610.3 |
| Yield (%) | 90.1 | 87.7 | 86.7 | 85.5 | 61.0 |
| Purity (%) | 99.7 | 99.7 | 99.9 | 99.9 | 99.9 |
| Water content (%) | 17.2 | 17.2 | 17.2 | 17.2 | 17.1 |

According to the above experimental results, it can be seen that: when the crystallization temperature is too low, the yield is higher, but more impurities are also precipitated, and single impurity content is greater than 0.1%. When the crystallization temperature is too high, the yield will be greatly reduced. Considering the above factors comprehensively, the selected crystallization temperature can be 5-25 °C, alternatively 10-25 °C, yet alternatively 15-25°C.

### Exploration of cooling gradients

**Table 9: Experimental examples of cooling gradient exploration**

| | Example 22 | Example 5 | Example 23 | Example 24 |
|---|---|---|---|---|
| The ratio of crude product to purified water (kg:L) | 1:4 | 1:4 | 1:4 | 1:4 |
| Dissolution temperature (°C) | 50 | 50 | 50 | 50 |
| Cooling gradient (°C) | 5 | 10 | 15 | 20 |
| Crystallization holding temperature (°C) | 20-25 | 20-25 | 20-25 | 20-25 |
| Crystallization time (h) | 8 | 8 | 8 | 8 |

**Table 10: Results of each experimental example of cooling gradient exploration**

| | Example 22 | Example 5 | Example 23 | Example 24 |
|---|---|---|---|---|
| Crystallization mass (g) | 883.7 | 855.5 | 848.6 | 857.4 |
| Yield (%) | 88.4 | 85.5 | 84.9 | 85.7 |
| Purity (%) | 99.9 | 99.9 | 99.8 | 99.7 |
| Water content (%) | 17.2 | 17.2 | 17.2 | 17.3 |

According to the above table, it can be seen that: when the cooling speed is too fast, the crystallization speed is fast, but impurities will be wrapped and precipitated quickly, resulting in reduced product purity. When the cooling speed is too slow, the crystallization time will be extended, and energy consumption and labor costs will be increased, while the yield does not change much. Therefore, taking all factors into consideration, the cooling rate can be 5-20 °C/h, alternatively 5-15 °C/h, yet alternatively 10°C/h.

### Example 25

1.0 kg of crude oxidized glutathione obtained in the examples of above (1) Synthesis of oxidized glutathione was added to 4.0 L of purified water at 40°C, and the mixture was stirred until the solid was dissolved. The mixture was filtered while hot, and then gradually cooled down to 20 °C, 10°C per hour. Finally, the mixture was controlled at a temperature of 15°C to 20°C and crystallized for 8 hours. The mixture was filtered. The filter cake was rinsed with ethanol, and dried to give 855.1 g of oxidized glutathione, with a one-step yield of 85.5% and a purity of 99.7%. The purity increase was 0.5%.

### Example 26

1.0 kg of crude oxidized glutathione obtained in the examples of above (1) Synthesis of oxidized glutathione was added to 4.0 L of purified water at 45°C, and 100 mL of 5% ferrous chloride solution was added. The mixture was stirred until the solid was dissolved. The mixture was filtered while hot, and then gradually cooled down to 20 °C, 10°C per hour. Finally, the mixture was controlled at a temperature of 15 °C to 20 °C, and crystallized for 7 hours. The mixture was filtered. The filter cake was rinsed with ethanol, and dried to give 849.2 g of oxidized glutathione, with a one-step yield of 84.9% and a purity of 99.9%. The purity increase was 0.7 %.

### Example 27

1.0 kg of crude oxidized glutathione obtained in the examples of above (1) Synthesis of oxidized glutathione was added to 5.0 L of purified water at 50°C, and the mixture was stirred until the solid was dissolved. The mixture was filtered while hot, and then gradually cooled down to 20 °C, 10°C per hour. Finally, the mixture was controlled at a temperature of 15 °C to 20 °C, and crystallized for 8 hours. The mixture was filtered. The filter cake was rinsed with ethanol, and dried to give 843.3 g of oxidized glutathione, with a one-step yield of 84.3 % and a purity of 99.7 %. The purity increase was 0.5 %.

### Example 28

1.0 kg of crude oxidized glutathione obtained in the examples of above (1) Synthesis of oxidized glutathione was added to 5.0 L of purified water at 50 °C, and 100 mL of 5% ferrous chloride solution was added. The mixture was stirred until the solid was dissolved. The mixture was filtered while hot, and then gradually cooled down to 20 °C, 10°C per hour. Finally, the mixture was controlled at a temperature of 15 °C to 20 °C, and crystallized for 8 hours. The mixture was filtered. The filter cake was rinsed with ethanol, and dried to give 848.6 g of oxidized glutathione, with a one-step yield of 84.9% and a purity of 99.9%. The purity increase was 0.7 %.

### Example 29

1.0 kg of crude oxidized glutathione obtained in the examples of above (1) Synthesis of oxidized glutathione was added to 4.0 L of purified water at 50 °C, and the mixture was stirred until the solid was dissolved. The mixture was filtered while hot, and then gradually cooled down to 20 °C, 10°C per hour. Finally, the mixture was controlled at a temperature of 20 °C to 25 °C, and crystallized for 6 hours. The mixture was filtered. The filter cake was rinsed with ethanol, and dried to give 851.6 g of oxidized glutathione, with a one-step yield of 85.2 % and a purity of 99.7 %. The purity increase was 0.5 %.

### Example 30

Preparation of impurity A: 4.6 g of Boc-Cys(Trt)-OH, 1.7 g of glycine tert-butyl hydrochloride and 7.6 g of HBTU were dissolved in 30 mL of DMF, and 2 mL of DIEA was added to the mixture dropwise. The reaction was carried out at room temperature for 24 hours, and then 100 mL of water was added. The mixture was extracted three times with ethyl acetate, and the organic phase was retained and dried over anhydrous magnesium sulfate. The solvent was evaporated to dryness, and then the residue was dissolved with 30 mL of dichloromethane. 30 mL of 30% trifluoroacetic acid aqueous solution was added to remove the protective groups such as Trt, Boc and tBu, etc. After the reaction was completed, the layers were separated and the aqueous layer was retained. 50 mL of ethanol was then added to the aqueous layer to precipitate a solid. The mixture was filtered with suction, and the filter cake was purified through preparative liquid phase chromatography to give Cys-Gly. Then reduced glutathione and Cys-Gly with a molar ratio of 1:1 were dissolved in 20 mL of water. 5 equivalents of DMSO was added into the mixture for oxidation. The reaction was carried out at room temperature for 10 hours, and then 40 mL of ethanol was added to precipitate a solid. The resulting solid was dissolved in 5 mL of 1% trifluoroacetic acid aqueous solution, and the mixture was separated by preparative liquid phase chromatography. The mobile phase was phase A: 1% trifluoroacetic acid/water; and phase B: 1% trifluoroacetic acid/acetonitrile. Finally, 2.0 g of impurity A was obtained by freeze-drying, with a total yield of 42% and a purity of 99.7%. ESI-MS [M + H]⁺ = 484.1.

### Example 31

Preparation of impurity B: 3.0 g of Boc-Glu-OtBu, 4.2 g of HCl·H-Cys(Trt)-OtBu and 7.6 g of HBTU were dissolved in 30 mL of DMF, and 3 mL of DIEA was added to the mixture dropwise. The reaction was carried out at room temperature for 18 hours, and then 100 mL of water was added. The mixture was extracted three times with ethyl acetate, and the organic phase was retained and dried over anhydrous magnesium sulfate. The solvent was evaporated to dryness, and then the residue was dissolved with 30 mL of dichloromethane. 40 mL of 30% trifluoroacetic acid aqueous solution was added to remove the protective groups such as Trt, Boc and tBu, etc. After the reaction was completed, the aqueous layer was separated. 50 mL of ethanol was then added to the aqueous layer to precipitate a solid. The mixture was filtered with suction, and the filter cake was purified through preparative liquid phase chromatography to give Glu-Cys. Then reduced glutathione and Glu-Cys with a molar ratio of 1:1 were dissolved in 20 mL of water. 5 equivalents of DMSO was added into the mixture for oxidation. The reaction was carried out at room temperature for 12 hours, and then 40 mL of ethanol was added to precipitate a solid. The resulting solid was dissolved in 5 mL of purified water, and the mixture was separated by preparative liquid phase chromatography. The mobile phase was phase A: 1% trifluoroacetic acid/water; and phase B: 1% trifluoroacetic acid/acetonitrile. Finally, 2.3 g of impurity B was obtained by freeze-drying, with a total yield of 41% and a purity of 99.7%. ESI-MS [M + H]⁺ = 555.2.

### Example 32

Preparation of impurity C: 3.1 g of reduced glutathione and 1.2 g of cysteine were dissolved in 15 mL of water, and 3.9 g of DMSO was added for oxidation. The mixture was reacted with stirring at room temperature for 9 hours. After the reaction was completed, 30 mL of ethanol was added to precipitate a solid. The resulting solid was dissolved in 5 mL of purified water, and the mixture was separated by preparative liquid phase chromatography. The mobile phase was phase A: 1% trifluoroacetic acid/water; and phase B: 1% trifluoroacetic acid/acetonitrile. Finally, 2.1 g of impurity C was obtained by freeze-drying, with a total yield of 49% and a purity of 99.8%. ESI-MS [M + H]⁺ = 427.1.

### (3) Crystal characterization and analysis

After testing, the melting point of the obtained crystal was 169°C (microscopic melting point tester X-5, manufacturer: Corey Instrument), which was different from the melting points of reported crystals, proving that the crystal was a new crystal form; the characteristic peaks (three high peaks and eight high peaks) obtained by X-ray powder diffraction (specification: D8 ADVANCE, manufacturer: Bruker Company, Germany, equipped with LynxEye detector, 20 scanning angle from 3° to 40°, scanning step size of 0.02°, scanning speed of 0.3s/step, and the light tube voltage and light tube current when measuring the sample were 40 KV and 40 mA, respectively) test were also inconsistent with reported crystal forms, and at the same time, its related peak shape proved that the crystal form was not a mixed crystal; after TG-DSC test (specification model: STA 449F5, manufacturer: NETZSCH, Germany), the weight loss of the sample from 50 to 180°C was measured through slow heating (1.0°C/min), and the water content of the obtained oxidized glutathione was 17.2 %, which was basically consistent with the water content (17.2%, a result of three parallel runs) of the sample measured by a Karl Fischer moisture meter (model: V20S, manufacturer: METTLER TOLEDO). The water content corresponds to the water content of oxidized glutathione heptahydrate calculated above, so the obtained crystals are determined to be oxidized glutathione heptahydrate.

Table 11 below shows the difference between the saturation solubility of crystals of oxidized glutathione heptahydrate and crystals of oxidized glutathione monohydrate and hexahydrate as described above.

**Table 11: Saturation solubility of oxidized glutathione hydrate**

| | Saturation solubility (g/L) <H₂O 30°C> |
|---|---|
| oxidized glutathione heptahydrate | 198.6 |
| oxidized glutathione hexahydrate | 173 |
| oxidized glutathione monohydrate | 13.5 |

At the same temperature, the solubility of heptahydrate in water is significantly better than that of monohydrate and is comparable to that of hexahydrate. Moreover, compared with the powdered crystals of the hexahydrate which are prone to generate static electricity and suffer a large amount of loss during a packaging process, the granular crystals of the obtained heptahydrate are more suitable for industrial production.

**Table 12: X-ray powder diffraction data of oxidized glutathione heptahydrate crystal form**

| Peak | Angle (2θ °) | D value (Å) | Intensity % |
|---|---|---|---|
| 1 | 7.433 | 11.884 | 2.9 |
| 2 | 8.238 | 10.724 | 100.0 |
| 3 | 9.750 | 9.065 | 11.0 |
| 4 | 10.619 | 8.325 | 15.6 |
| 5 | 12.459 | 7.099 | 3.0 |
| 6 | 14.151 | 6.254 | 3.3 |
| 7 | 14.757 | 5.998 | 3.0 |
| 8 | 16.338 | 5.421 | 80.5 |
| 9 | 17.820 | 4.973 | 6.4 |
| 10 | 19.539 | 4.540 | 25.5 |
| 11 | 20.211 | 4.390 | 5.4 |
| 12 | 21.230 | 4.182 | 4.5 |
| 13 | 21.621 | 4.107 | 3.5 |
| 14 | 22.738 | 3.908 | 11.3 |
| 15 | 23.200 | 3.831 | 10.7 |
| 16 | 24.551 | 3.623 | 33.6 |
| 17 | 25.024 | 3.556 | 5.1 |
| 18 | 26.806 | 3.323 | 25.6 |
| 19 | 28.414 | 3.139 | 3.8 |
| 20 | 28.982 | 3.078 | 3.1 |
| 21 | 29.351 | 3.041 | 5.3 |
| 22 | 32.021 | 2.793 | 6.1 |
| 23 | 32.895 | 2.721 | 7.7 |
| 24 | 34.053 | 2.631 | 8.9 |
| 25 | 34.618 | 2.589 | 17.5 |
| 26 | 36.699 | 2.447 | 8.1 |

In summary, the present disclosure provides a method for preparing oxidized glutathione and its new crystal form and impurities, which simplifies the synthesis method of oxidized glutathione. The synthesis of impurities provides a guarantee for the subsequent quality study of oxidized glutathione. At the same time, the use of purified water to recrystallize the synthesized oxidized glutathione not only obtains high-purity oxidized glutathione, but also simplifies the existing purification method. Compared with the existing crystal forms, the new crystal form of the newly discovered heptahydrate has a simpler synthesis method, better water solubility, and better stability, and is more suitable for the industrial production of oxidized glutathione.

## Claims

1. A crystal of a heptahydrate of a compound of formula (I):

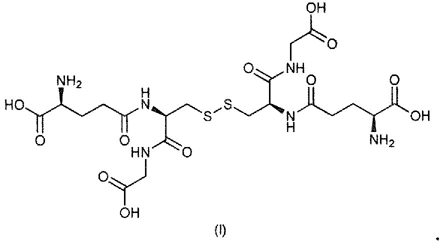

2. The crystal of claim 1, which is **characterized by** an X-ray powder diffraction pattern obtained using CuKα radiation having at least the following characteristic peaks at °2Θ: 8.238±0.2, 16.338±0.2 and 24.551±0.2.

3. The crystal of claim 2, which is **characterized by** an X-ray powder diffraction pattern obtained using CuKa radiation having at least the following characteristic peaks at °2Θ: 10.619±0.2, 19.539±0.2, 26.806±0.2 and 34.618±0.2.

4. The crystal of claim 3, which is **characterized by** an X-ray powder diffraction pattern obtained using CuKα radiation having at least the following characteristic peaks at °2Θ: 9.750±0.2, 22.738±0.2 and 23.200±0.2.

5. The crystal of claim 1, which is **characterized by** having an X-ray powder diffraction pattern substantially as shown in Fig. 5.

6. The crystal of any one of claims 1 to 5, which is further **characterized by** having a melting point of 169 ± 2 °C.

7. The crystal of any one of claims 1 to 6, which is further **characterized by** a weight loss of about 14 ± 1% at 50-100 °C, and a weight loss of about 3 ± 1% at 100-160 °C in thermogravimetric analysis.

8. The crystal of any one of claims 1 to 7, which is further **characterized by** having a thermogravimetric diagram as shown in Fig. 6.

9. A method for preparing a compound of formula (1), 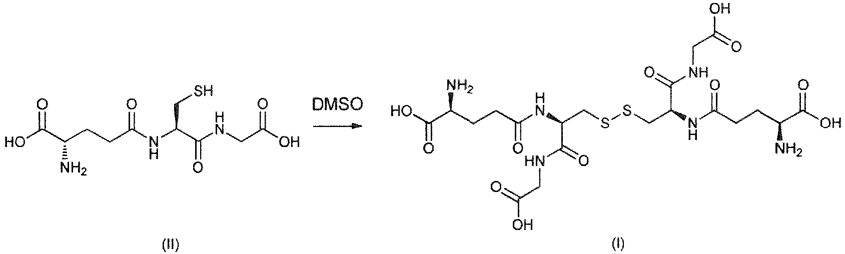 comprising oxidizing the compound of formula (II) with DMSO to obtain the compound of formula (I).

10. The method of claim 9, wherein the molar ratio of DMSO to the compound of formula (II) is 2:1 to 25:1, alternatively 2.5:1 to 5:1, yet alternatively 2:1, 2.5:1, 3:1, 3.5:1, 4:1, 4.5:1 or 5:1.

11. The method of claim 9 or 10, wherein a polar solvent is additionally added to the reaction to promote the dissolution of the compound of formula (II); optionally, the polar solvent is selected from the group consisting of water, formamide, trifluoroacetic acid, acetonitrile, DMF, hexamethylphosphoramide, methanol, ethanol, acetic acid, isopropanol, pyridine, tetramethylethylenediamine, acetone, triethylamine, n-butanol, dioxane, tetrahydrofuran, methyl formate, tributylamine, methyl ethyl ketone, ethyl acetate, chloroform, trioctylamine, dimethyl carbonate and diethyl ether, or a compound thereof, yet alternatively water.

12. The method of claim 11, wherein the ratio of the polar solvent to the compound of formula (II) is 200 to 2000 mL, alternatively 250 mL to 1000 mL, yet alternatively 250 mL, 300 mL, 500 mL, 750 mL or 1000 mL of the polar solvent per 100 g of the compound of formula (II).

13. The method of any one of claims 9 to 12, wherein the pH of the reaction is adjusted to 2 to 9, alternatively 3 to 7, yet alternatively 5 to 7, such as 5, 5.5, 6, 6.5, 7, 8 or 9.

14. The method of any one of claims 9 to 13, wherein the reaction is carried out at -10 °C to 60°C, alternatively 5 °C to 30 °C, such as 25 °C, 40 °C or room temperature.

15. The method of any one of claims 9 to 14, wherein the reaction is carried out for 5 to 60 h, alternatively 10 to 48 h, such as 10 h, 15 h, 20 h, 25 h, 30 h, 35h, 40h or 45h.

16. The method of any one of claims 9 to 15, wherein after the reaction is completed, the pH is adjusted to the isoelectric point of oxidized glutathione (pH 2.75 to 2.90), and stirring crystallization is performed for at least 5 h, alternatively at least 10 hours.

17. The method of claim 16, wherein the stirring crystallization is performed at 5°C to 40 °C, alternatively 5°C, 10 °C, 15 °C, 20 °C or room temperature.

18. The method of any one of claims 9 to 17, wherein the purity of the compound of formula (I) obtained is ≥ 98%, alternatively ≥ 98.5%, alternatively ≥ 99%, alternatively ≥ 99.7%, alternatively ≥ 99.8%, alternatively ≥ 99.9%; and wherein the total content of impurity A, impurity B and impurity C in the product is less than 1%, alternatively less than 0.5%, alternatively less than 0.3%, alternatively less than 0.1%,
| | |
|---|---|
| Impurity A | 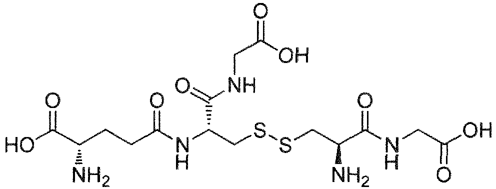 |
| Impurity B | 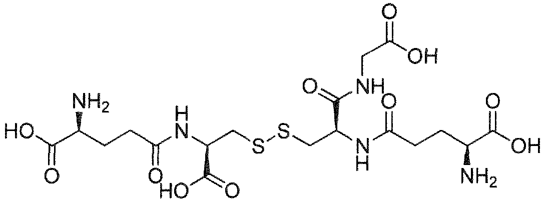 |
| Impurity C | 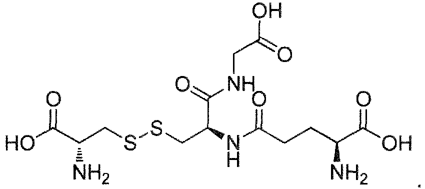 |

19. A method for refining the oxidized glutathione of any one of claims 9 to 18, comprising:
1) dissolving a crude oxidized glutathione in purified water, wherein the amount of purified water is 3 to 5 times the mass of the crude oxidized glutathione;
2) filtering the solution while hot after the dissolution;
3) cooling the filtrate to 10 to 25°C, alternatively 12 to 20°C for crystallization.

20. The method of claim 19, wherein in step 1), the temperature of the purified water is 35°C to 55°C, alternatively 40°C to 50°C; and optionally, ferrous chloride solution is added to remove residual raw materials, alternatively, 100 mL of 5% ferrous chloride solution is added per kilogram of oxidized glutathione.

21. The method of claim 19 or 20, wherein in step 3), the crystallization time of recrystallization is 3 to 10 hours, alternatively 4 to 6 hours; optionally, gradient cooling is adopted, and the cooling rate is 10°C per hour.

22. A method for preparing the crystal of oxidized glutathione heptahydrate according to any one of claims 1 to 8, comprising recrystallizing the oxidized glutathione prepared according to any one of claims 9 to 21 in purified water; optionally, the method comprises the following steps:
1) dissolving the oxidized glutathione in purified water and stirring until dissolved;
2) filtering the solution while hot after the dissolution, and then gradually cooling down to the target temperature;
3) crystallization under controlled temperature.

23. The method of claim 22, wherein in step 1), 2 L to 6 L, alternatively 3 L to 6 L, yet alternatively 3 L to 5 L, still alternatively 4 L of purified water is used per kilogram of oxidized glutathione.

24. The method of claim 22 or 23, wherein in step 1), the temperature of the purified water is 40-60°C, alternatively 40-50 °C, yet alternatively 50 °C during the dissolution process.

25. The method of any one of claims 22 to 24, wherein in step 1), ferrous chloride solution is added to better remove residual raw materials, optionally, 100 mL of 5% ferrous chloride solution is added per kilogram of oxidized glutathione.

26. The method of any one of claims 22 to 25, wherein in step 2), the cooling gradient is 5-25 °C/h, alternatively 5-20 °C/h, alternatively 5-15 °C/h, yet alternatively 10 °C/h.

27. The method of any one of claims 22 to 26, wherein in step 2), the target temperature is 5-25 °C, alternatively 10-25 °C, yet alternatively 15-25 °C.

28. The method of any one of claims 22 to 27, wherein in step 3), the temperature is controlled at 5-25 °C, alternatively 10-25 °C, yet alternatively 15-25 °C.

29. The method of any one of claims 22 to 28, wherein in step 3), the crystallization time is 6 to 12 hours, alternatively 6 to 8 hours.

30. A compound selected from:
| | |
|---|---|
| Impurity A | 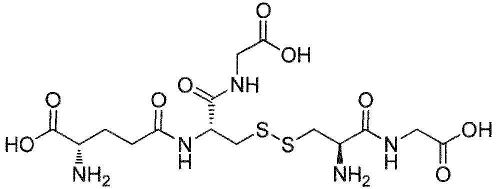 |
| Impurity B | 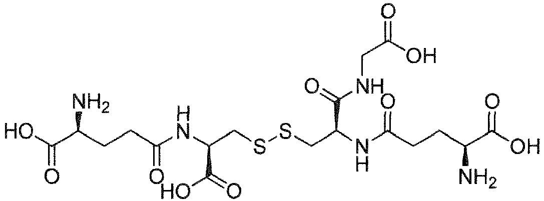 |
| Impurity C | 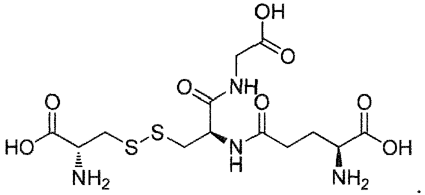 |

31. A method for preparing impurity A in claim 30, 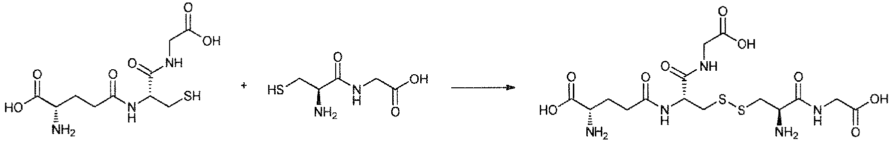 comprising oxidizing reduced glutathione and Cys-Gly in a molar ratio of 1:1 with DMSO.

32. The method of claim 31, wherein the amount of DMSO is 2 to 10 equivalents, alternatively 3 to 5 equivalents.

33. The method of claim 31 or 32, comprising the steps of:
1) condensing Boc-Cys(Trt)-OH and glycine tert-butyl ester in a molar ratio of 1:1 to 1:3 under the catalysis of 1.5 to 4 equivalents of a condensation agent (such as HATU, HBTU, PyBOP, DEPBT, etc., alternatively HBTU and DEPBT) and 2 equivalents of an organic tertiary amine (such as N,N-diisopropylethylamine and triethylamine, alternatively N,N-diisopropylethylamine), wherein the reaction is carried out in DMF or dichloromethane;
2) removing Trt, Boc and tBu protective groups with trifluoroacetic acid to obtain Cys-Gly;
3) oxidizing reduced glutathione and Cys-Gly in a molar ratio of 1:1 with 3 to 5 equivalents of DMSO.

34. A method for preparing impurity B in claim 30, 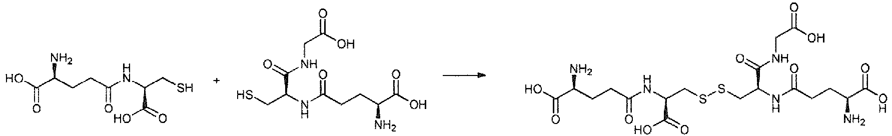 comprising oxidizing reduced glutathione and Glu-Cys in a molar ratio of 1:1 with DMSO.

35. The method of claim 34, wherein the amount of DMSO is 2 to 10 equivalents, alternatively 3 to 5 equivalents.

36. The method of claim 34 or 35, comprising the steps of
1) condensing Boc-Glu-OtBu and H-Cys(Trt)-OtBu in a molar ratio of 1:1 to 1:2 under the catalysis of 1.5 to 4 equivalents of a condensation agent (such as HATU, HBTU, PyBOP, DEPBT, etc., alternatively HBTU and DEPBT) and 2 equivalents of an organic tertiary amine (such as N,N-diisopropylethylamine and triethylamine, alternatively N,N-diisopropylethylamine),
2) removing Boc, Trt, and tBu protective groups with trifluoroacetic acid to obtain Glu-Cys;
3) oxidizing reduced glutathione and Glu-Cys in a molar ratio of 1:1 with 3 to 5 equivalents of DMSO.

37. A method for preparing impurity C in claim 30, comprising oxidizing reduced glutathione and cysteine in a molar ratio of 1:1 with DMSO.

38. The method of claim 37, wherein the amount of DMSO is 2 to 10 equivalents, alternatively 3 to 5 equivalents.
